Europäisches Patentamt

European Patent Office

Office européen des brevets

(19)

(11) Veröffentlichungsnummer : **0 451 261 B1**

(12) **EUROPÄISCHE PATENTSCHRIFT**

(45) Veröffentlichungstag der Patentschrift :
**15.09.93 Patentblatt 93/37**

(51) Int. Cl.$^5$ : **A61K 7/135,** A61K 7/00,
A45D 24/10

(21) Anmeldenummer : **90917554.9**

(22) Anmeldetag : **26.10.90**

(86) Internationale Anmeldenummer :
**PCT/EP90/01917**

(87) Internationale Veröffentlichungsnummer :
**WO 91/06279 16.05.91 Gazette 91/11**

(54) **MITTEL UND VERFAHREN ZUM BLEICHEN VON HAAREN UNTER LICHTEINWIRKUNG.**

(30) Priorität : **01.11.89 GB 8924581**

(43) Veröffentlichungstag der Anmeldung :
**16.10.91 Patentblatt 91/42**

(45) Bekanntmachung des Hinweises auf die
Patenterteilung :
**15.09.93 Patentblatt 93/37**

(84) Benannte Vertragsstaaten :
**DE ES FR GB IT**

(73) Patentinhaber : **Wella Aktiengesellschaft**
**Berliner Allee 65**
**D-64295 Darmstadt (DE)**

(72) Erfinder : **GODFREY, Robin, Edward, Dr.**
**7 Copse Hill Welwyn**
**Herts (GB)**
Erfinder : **CLAUSEN, Thomas, Dr.**
**Ernst-Pasqué-Strasse 35A**
**D-6146 Alsbach (DE)**
Erfinder : **BALZER, Wolfgang, R., Dr.**
**Im Kiessling 12**
**D-6146 Alsbach (DE)**
Erfinder : **Mahal, Georg, Dr.**
**Uferweg 20**
**D-6460 Gelnhausen (DE)**
Erfinder : **Rau, Robert, Dr.**
**Schillerstrasse 49 B**
**D-6112 Gross-Zimmern (DE)**

EP 0 451 261 B1

## Beschreibung

Gegenstand der vorliegenden Erfindung ist ein Verfahren zum Bleichen von Haaren, bei dem das zuvor mit einem einen optischen Photosensibilisator enthaltenden Mittel behandelte Haar unter Lichteinwirkung gebleicht wird, sowie Mittel zum Bleichen der Haare unter Lichteinwirkung.

Wie schon lange bekannt ist, werden menschliche Haare vom Sonnenlicht gebleicht. Der Grad der erzielbaren Aufhellung des Haares ist dabei von der ursprünglichen Haarfarbe und von der Einwirkungsdauer des Sonnenlichtes abhängig.

Da es jedoch auch bei intensiver Sonnenstrahlung in der Regel mehrere Tage dauert, bis eine sichtbare Bleichwirkung durch das Sonnenlicht erzielt wird, ist dieses Verfahren zum gezielten Bleichen nicht geeignet.

Dem Schutz der Haare vor der bleichenden Wirkung des Sonnenlichts dient ein aus der EP-OS 0 193 932 bekanntes Haarbehandlungsmittel, welches eine Kombination üblicher UV-Filtersubstanzen mit Alkohol enthält.

Aus der US-PS 4 792 341 ist ein Verfahren zum Bleichen von Haaren bekannt, bei dem das Haar einem oder mehreren Strahlungsstößen künstlichen Lichts ausgesetzt wird, wobei die Lichtenergie ausreichend ist, um das Melanin im Haar zu bleichen. Das aus der US-PS 4 792 341 bekannte Verfahren beruht auf der durch die Verwendung langwelliger Strahlung mit hoher Intensität bewirkten thermischen Zersetzung des Melanins.

Die Intensität der auf das Haar auftreffenden Strahlung muß in diesem Verfahren sehr hoch gewählt werden, so daß eine starke Erhitzung des Haares beobachtet wird, wodurch eine Schädigung des Haares auftreten kann.

Derzeit wird das Bleichen des Haares üblicherweise durch Einwirkung von einem oxidationsmittelhaltigen, insbesondere wasserstoffperoxidhaltigen, Bleichmittel auf das Haar bewirkt, wobei eine Verstärkung der Bleichwirkung durch Zusätze von Salzen der Persäuren erreicht werden kann.

Aus der US-PS 3 816 615 ist ein alkalisches, peroxidhaltiges Haarbleichmittel für Haar bekannt, das zur Verstärkung der Bleichwirkung bestimmte Dicarbonylverbindungen enthält.

Das der modischen Veränderung des natürlichen Haarfarbtones dienende Bleichen des Haares basiert chemisch auf einer, zum Beispiel oxidativen, Veränderung des Haarpigmentes  Melanin.

Obwohl das Melanin im Haar nur in relativ geringen Mengen vorhanden ist, muß beim Bleichen von Haaren mit den üblichen wasserstoffperoxidhaltigen Bleichmitteln mit einem großen Überschuß an Wasserstoffperoxid im alkalischen pH-Bereich gearbeitet werden, um eine ausreichende Bleichwirkung zu garantieren.

Bei diesem derzeit üblichen Verfahren zum Bleichen von Haaren ist aufgrund des verwendeten Wasserstoffperoxidüberschusses und des alkalischen pH-Wertes eine Haarschädigung nicht zu vermeiden.

Es bestand die Aufgabe, eine Möglichkeit zu schaffen, mit der ein schonenderes Bleichen von Haaren mit Licht gelingt. Ein neues Verfahren sollte auf photochemischen und nicht auf photothermischen Reaktionen beruhen, um ein haarschädigendes Aufheizen des Haares, wie es bei dem aus der US-PS 4 792 341 bekannten Verfahren vorkommt, zu vermeiden. Zudem sollte das neue Verfahren durch photochemische Erzeugung einer auf die Melaninkonzentration des Haares abgestimmten Konzentration an Oxidationsmittel in der Lage sein, das Haar ohne einen Überschuß an Oxidationsmittel in einem angemessenen Zeitraum zu bleichen.

Es wurde nun gefunden, daß durch ein Verfahren zum Bleichen der Haare unter Lichteinwirkung, bei dem man

a) zunächst 10 bis 120 g eines in Form einer Lösung, einer Emulsion oder eines Gels vorliegenden, mindestens einen optischen Photosensibilisator und eine Verbindung, die in der Lage ist, ein Wasserstoff-Radikal zur Verfügung zu stellen, enthaltenden Mittels auf die Haare aufträgt und bis zu 60, vorzugsweise 5 bis 30, Minuten lang einwirken läßt,

b) sodann das Haar mit sichtbarem Licht und/oder UV-Licht einer Wellenlänge oder eines Wellenlängenbereichs, das in der Lage ist, den enthaltenen optischen Photosensibilisator zu aktivieren, mit einer für das Bleichen des Haares ausreichenden Intensität bestrahlt,

c) anschließend das Haar gründlich spült und sodann trocknet,

die gestellte Aufgabe in hervorragender Weise gelöst wird.

Das neue Verfahren zum Bleichen von Haaren ist besonders haarschonend, da es aufgrund der in-situ-Erzeugung von Wasserstoffperoxid im Haar in der Lage ist, ohne die Verwendung von Wasserstoffperoxidüberschüssen eine gute Bleichwirkung zu erzielen. Der Bleichvorgang läßt sich zudem, wenn der gewünschte Grad der Aufhellung erreicht ist, sofort beenden, indem die Lichtquelle abgeschaltet wird. Durch das neue Verfahren zum Bleichen der Haare ist der gewünschte Grad der Aufhellung wesentlich besser steuerbar, als bei dem bisher üblichen Verfahren zum Bleichen der Haare unter Verwendung von wasserstoffperoxidhaltigen Mitteln.

Die in-situ-Erzeugung von Wasserstoffperoxid wird durch den optischen Photosensibilisator ermöglicht, der in das Haar eindringt und während der Bestrahlung mit Licht mit Luftsauerstoff und einer Verbindung, die

2

in der Lage ist, ein Wasserstoff-Radikal zur Verfügung zu stellen, insbesondere Wasser oder einem Alkohol, eine zum Bleichen des Melanins ausreichende Menge an Wasserstoffperoxid erzeugt.

Im Gegensatz zu dem aus der US-PS 4 792 341 bekannten photothermischen Verfahren zum Bleichen der Haare, das ein starkes Aufheizen des Haares und eine daraus resultierende Haarschädigung zur Folge hat, beruht das erfindungsgemäße Verfahren auf der photochemischen Aktivierung eines vor der Bestrahlung auf das Haar aufgebrachten optischen Photosensibilisators und der durch diesen bewirkten Erzeugung von Wasserstoffperoxid im Haar. Die im erfindungsgemäßen Verfahren verwendeten Lichtintensitäten sind daher erheblich kleiner. Zudem erfolgt die Bestrahlung des Haares bevorzugt im kontinuierlichen Modus.

Die im ersten Verfahrensschritt des erfindungsgemäßen Verfahrens aufgetragene Menge des mindestens einen optischen Photosensibilisator enthaltenden Mittels soll, wenn das Mittel in Form einer Lösung vorliegt, besonders bevorzugt 15 bis 40 g, wenn das Mittel in Form einer Emulsion oder eines Gels vorliegt, besonders bevorzugt 40 bis 80 g, betragen.

In einer besonderen Ausführungsform des erfindungsgemäßen Verfahrens wird das Haar vor dem Auftragen des den optischen Photosensibilisator enthaltenden Mittels mit Wasser angefeuchtet.

Der in den beiden hier beschriebenen erfindungsgemäßen Verfahren zum Bleichen der Haare unter Lichteinwirkung verwendeten Mittel enthaltene optische Photosensibilisator ist bevorzugt ein Keton und kann beispielsweise ausgewählt sein aus Benzophenon, 3-Brom-benzophenon, 4-Brom-benzophenon, 2,2',4,4'-Tetrahydroxybenzophenon, 2,5-Dihydroxyacetophenon, Benzil, p-Benzochinon, 2,3-Butandion, Michlers Keton, Acetophenon, 4-Benzoylbenzoesäure, Methoxyacetophenon und 9-Fluorenon.

Gegenstand der vorliegenden Erfindung ist weiterhin ein Mittel zum Bleichen der Haare unter Lichteinwirkung zur Verwendung nach dem vorstehend beschriebenen Verfahren in Form einer Lösung, einer Emulsion oder eines Gels mit einem Gehalt an mindestens einer Verbindung, die in der Lage ist, ein Wasserstoff-Radikal zur Verfügung zu stellen, welches dadurch gekennzeichnet ist, daß es frei von Peroxiden ist und mindestens einen optischen Photosensibilisator enthält, der ausgewählt ist aus Benzophenon, 3-Brombenzophenon, 4-Brombenzophenon, 2,5-Dihydroxy-acetophenon, Benzil, p-Benzochinon, 2,3-Butandion, Michlers Keton, Acetophenon, 4-Benzylbenzoesäure, Methoxyacetophenon und 9-Fluorenon.

Von den vorstehend genannten optischen Photosensibilisatoren enthält das erfindungsgemäße Mittel zum Bleichen der Haare unter Lichteinwirkung bevorzugt Benzophenon.

Das erfindungsgemäße Mittel enthält bevorzugt 0,1 bis 10 Gewichtsprozent, besonders bevorzugt 0,5 bis 5 Gewichtsprozent, mindestens eines optischen Photosensibilisators.

Der in dem erfindungsgemäßen Mittel enthaltene optische Photosensibilisator ist bevorzugt ein nichtionisches Molekül, das aufgrund seiner geringen Größe in der Lage ist, in das Haar einzudringen und das sich nach dem Bleichen leicht wieder ausspülen läßt.

Der in dem erfindungsgemäßen Mittel enthaltene optische Photosensibilisator soll bevorzugt ein Triplettsensibilisator sein, das heißt, der durch die Lichtabsorption aktivierte Molekülzustand des optischen Photosensibilisators ist ein Triplettzustand, dessen Energie strahlungslos an ein Akzeptormolekül abgegeben wird.

Der in den Triplettzustand angeregte optische Photosensibilisator kann seine Energie zum einen auf das Akzeptormolekül Sauerstoff übertragen, aus dem dann über die Bildung von Singulettsauerstoff und Wasserstoffabstraktion Wasserstoffperoxid entsteht.

Andererseits kann der in den Triplettzustand angeregte optische Photosensibilisator jedoch auch Wasserstoff aus dem Lösungsmittel abstrahieren und über einen noch nicht völlig geklärten Mechanismus, in dessen Verlauf Wasserstoffperoxid gebildet wird, die Oxidation des Melanins des Haares bewirken. Die Vielzahl chemischer Reaktionen, die in derartigen Photooxidationen von Bedeutung sein können, sind zum Beispiel bei C.S. Foote, Science (1968), Vol. 162, Seiten 963 bis 970 beschrieben.

Die Bedeutung der beiden konkurrierenden Reaktionsmechanismen des optischen Photosensibilisators für das hier beschriebene Verfahren zum Bleichen von Haaren unter Lichteinwirkung hängt von den jeweiligen Reaktionsbedingungen ab. Der angeregte Triplettzustand des optischen Photosensibilisators soll mit hoher Quantenausbeute gebildet werden und ausreichend Energie sowie eine ausreichende Lebensdauer besitzen, um durch die Reaktion mit dem Sauerstoff der Luft oder einem geeigneten Lösungsmittel die Bildung von Wasserstoffperoxid im Haar zu gewährleisten.

Das erfindungsgemäße Mittel zum Bleichen der Haare unter Lichteinwirkung soll bevorzugt einen optischen Photosensibilisator enthalten, der durch sichtbares Licht und/oder UV-Licht, besonders bevorzugt jedoch sichtbares Licht, aktiviert werden kann.

Die in dem hier beschriebenen Mittel zum Bleichen der Haare enthaltene Verbindung, die in der Lage ist, ein Wasserstoff-Radikal zur Verfügung zu stellen, kann beispielsweise Wasser sein, ist jedoch bevorzugt ein Alkohol, beispielsweise Ethanol, Methanol, Isopropanol, Cyclopentanol, Benzylalkohol, Cyclohexanol oder Glykol. Sie ist mindestens in einer Menge von 0,1 Gewichtsprozent enthalten.

Die Zubereitungsform des erfindungsgemäßen Mittels zum Bleichen der Haare unter Lichteinwirkung ist bevorzugt die einer Lösung, insbesondere die einer alkoholischen oder wäßrig-alkoholischen Lösung. Die Zubereitungsform kann jedoch auch die einer Creme, eines Gels oder einer Emulsion sein. Das Mittel kann auch im Gemisch mit einem Treibgas oder mittels einer Pumpe versprüht werden.

Übliche Zusätze in Lösungen, Cremes, Emulsionen oder Gelen sind, neben Lösungsmitteln wie Wasser und niederen aliphatischen Alkoholen, beispielsweise Ethanol, Propanol und Isopropanol oder Glykolen wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Das erfindungsgemäße Mittel muß frei von in der Kosmetik üblichen Radikalfängern, wie Tocopherol und Ascorbinsäure oder deren Fettsäureester oder β-Carotin und in der Kosmetik üblichen UV-Filtersubstanzen, sofern diese nicht als optische Photosensibilisatoren für das erfindungsgemäße Verfahren geeignet sind, sein.

Die im zweiten Verfahrensschritt des zuvor beschriebenen erfindungsgemäßen Verfahrens verwendbare Lichtquelle soll Licht einer Wellenlänge oder eines Wellenlängenbereichs des sichtbaren Lichts und/oder des UV-Lichts abstrahlen, das den im zuvor beschriebenen erfindungsgemäßen Mittel zum Bleichen der Haare enthaltenen optischen Photosensibilisator aktivieren kann.

Im zweiten Verfahrensschritt des vorstehend beschriebenen erfindungsgemäßen Verfahrens zum Bleichen der Haare wird das Haar bevorzugt mit Licht einer Lichtquelle, die ausgewählt ist aus Lichtbogenlampen, einer Deuterium-Lampe, einer Quecksilberdampf-Lampe oder einer Xenon-Lampe, und Lasern, wie zum Beispiel einem Halbleiterlaser, einem Stickstoff-Laser oder einem Helium-Cadmium-Laser, bestrahlt, jedoch sind auch andere Lichtquellen, wie zum Beispiel die Sonne, für das erfindungsgemäße Verfahren geeignet, sofern sie die für die Aktivierung des jeweiligen optischen Photosensibilisator geeignete Wellenlänge beziehungsweise den geeigneten Wellenlängenbereich in einer ausreichenden Intensität abstrahlen können.

Wird im Verfahrensschritt (b) des vorstehend beschriebenen erfindungsgemäßen Verfahrens zur Bestrahlung das Licht einer künstlichen Lichtquelle verwendet, soll diese bevorzugt im kontinuierlichen Modus betrieben werden.

Im Verfahrensschritt(b) des erfindungsgemäßen Verfahrens kann das Licht einer geeigneten Lichtquelle, zum Beispiel durch Lichtleitfasern, in einen an den Innenseiten der Kammzinken Licht abstrahlenden Lichtkamm eingespeist werden.

Die im Verfahrensschritt (b) verwendete Lichtquelle kann auch innerhalb einer für UV-Licht undurchlässigen Haube angeordnet sein, die, ähnlich einer Trockenhaube, über das Haar gestülpt wird, jedoch im Unterschied zur herkömmlichen Trockenhaube, am Haubenrand einen den Lichtaustritt verhindernden Abschluß, zum Beispiel in Form einer Manschette, aufweist.

Die für UV-Licht undurchlässige, vorzugsweise innen verspiegelte, Haube kann ferner mit einem Gebläse zur Abführung von Wärme und/oder zur Bewegung der Haare und/oder zur Haartrocknung ausgestattet sein.

Das Haar soll im Verfahrensschritt (b) des vorstehend beschriebenen erfindungsgemäßen Verfahrens mit sichtbarem und/oder UV-Licht bestrahlt werden, dessen auf das Haar auftreffende Intensität ohne die Verwendung des optischen Photosensibilisators nicht ausreichend wäre, um das Haar innerhalb der in Betracht kommenden Bestrahlungsdauer zu bleichen. Die für das Bleichen der Haare nach dem vorstehend beschriebenen Verfahren benötigte Bestrahlungsdauer hängt, neben anderen Faktoren, wie zum Beispiel dem gewünschten Grad der Aufhellung, auch von der Art der Lichtquelle sowie der Bestrahlungsintensität ($W/cm^2$) ab. Die Bestrahlungsdauer bei dem erfindungsgemäßen Verfahren liegt zwischen 1 Sekunde bei Verwendung des Lichtkamms, und 2 Tagen, wenn im Verfahrensschritt (b) mit Sonnenlicht bestrahlt wird, bevorzugt beträgt die Bestrahlungsdauer jedoch 10 bis 120 Minuten.

Ist der gewünschte Grad der Aufhellung durch die Behandlung des Haares nach dem erfindungsgemäßen Verfahren erreicht, kann das Haar gegebenenfalls, bevor das den optischen Photosensibilisator enthaltene Mittel ausgespült wird, mit einem Nachbehandlungsmittel behandelt werden, das einen Stoff enthält, der in der Lage ist, den optischen Photosensibilisator zu desaktivieren. Dieser Stoff kann zum Beispiel ein Triplett-Quencher wie beispielsweise β-Carotin sein, der den angeregten Triplettzustand des optischen Photosensibilisators desaktiviert. In dem Nachbehandlungsmittel kann jedoch auch ein Stoff eingesetzt werden, der den optischen Photosensibilisator chemisch so verändert, daß dieser für das erfindungsgemäße Verfahren zum Bleichen von Haaren unter Lichteinwirkung nicht mehr wirksam ist.

Zum Ausspülen des Haares im Verfahrensschritt (c) wird eine Flüssigkeit verwendet, in der der optische Photosensibilisator löslich ist. Im Falle von Benzophenon wird vorzugsweise Ethanol oder eine wäßrig-alkoholische Lösung verwendet. Durch die Entfernung, beziehungsweise die Desaktivierung des optischen Photosensibilisators soll eine mögliche weitere Bleichung des Haares durch natürliche oder künstliche Lichtquellen, zum Beispiel Sonne oder Sonnenbank, ausgeschlossen werden.

Das erfindungsgemäße Verfahren ist zum Bleichen des gesamten menschlichen Kopfhaares geeignet. Es kann jedoch auch zum Bleichen von Haarsträhnen, Haarteilen und Perücken sowie zum Bleichen tierischer Haare, beispielsweise von Pelzen, angewandt werden.

Das erfindungsgemäße Verfahren zum Bleichen der Haare ist insbesondere zur Erzeugung heller Strähnen im Haar geeignet.

Das Einarbeiten heller Strähnen in das Haar kann nach dem erfindungsgemäßen Verfahren, zum Beispiel unter Verwendung von Strähnen-Folienhauben, erfolgen.

In einer besonderen Ausführungsform des erfindungsgemäßen Verfahrens wird das Haar mit einer an sich bekannten Strähnen-Folienhaube, welche vorzugsweise so ausgewählt ist, daß sie für UV-Licht undurchlässig ist, bedeckt, anschließend wird die Haube mit einer, vorzugsweise dünnen, Häkelnadel mehrmals durchstochen, die jeweils darunterliegenden Haarsträhnen werden erfaßt und durch die Folie herausgezogen. Sodann werden 50 bis 100 g des erfindungsgemäßen Mittels auf die Haarsträhnen aufgetragen. Nach einer Einwirkungszeit von bis zu 60 Minuten, vorzugsweise 5 bis 30 Minuten, werden die herausgezogenen Strähnen mit Licht einer Wellenlänge oder eines Wellenlängenbereichs, das in der Lage ist, den in dem erfindungsgemäßen Mittel enthaltenen optischen Photosensibilisator zu aktivieren, mit einer für das Bleichen des Haares ausreichenden Intensität bestrahlt. Anschließend wird die Strähnen-Folienhaube entfernt, das Haar mit einem zum Lösen des optischen Photosensibilisators geeigneten Lösungsmittel gespült und sodann getrocknet.

In einer anderen Ausführungsform des erfindungsgemäßen Verfahrens wird das, gegebenenfalls mit Wasser angefeuchtete, Haar zunächst mit 10 bis 120 g des erfindungsgemäßen Mittels durchtränkt, sodann mit einer für UV-Licht undurchlässigen Strähnen-Folienhaube bedeckt, die Haube mit einer, vorzugsweise dünnen, Häkelnadel mehrmals durchstochen, die jeweils darunterliegenden Haarsträhnen erfaßt, durch die Folie herausgezogen und sodann die herausgezogenen Strähnen mit Licht einer Wellenlänge oder eines Wellenlängenbereichs, das in der Lage ist, den in dem erfindungsgemäßen Mittel enthaltenen optischen Photosensibilisator zu aktivieren, mit einer für das Bleichen des Haares ausreichenden Intensität bestrahlt. Anschließend wird die Strähnen-Folienhaube entfernt, das Haar gründlich mit einem zum Lösen des optischen Photosensibilisators geeigneten Lösungsmittel gespült und getrocknet.

Die Verwendung einer Strähnen-Folienhaube, die für UV-Licht undurchlässig ist, insbesondere deren Oberfläche strahlungsreflektierend ist, bietet die Möglichkeit, die Kopfhaut vor der Lichteinwirkung zu schützen. Durch die reflektierende Oberfläche werden die Erwärmung der Strähnen-Folienhaube und die Lichtverluste vermindert.

In einer weiteren Ausführungsform des erfindungsgemäßen Verfahrens wird das, gegebenenfalls mit Wasser vorgefeuchtete, Haar mit einer für UV-Licht undurchlässigen, beispielsweise verspiegelten, Strähnen-Folienhaube bedeckt, anschließend wird die Haube mit einer, vorzugsweise dünnen, Häkelnadel mehrmals durchstochen, die jeweils darunterliegenden Haarsträhnen werden erfaßt und durch die Folie herausgezogen. Sodann werden 50 bis 100 g des erfindungsgemäßen Mittels, auf die Haarsträhnen aufgetragen. Nach einer Einwirkungszeit von bis zu 60 Minuten, vorzugsweise 5 bis 30 Minuten, wird eine für UV-Licht undurchlässige Haube über den Kopf gestülpt, in der eine Lichtquelle, welche Licht einer Wellenlänge oder eines Wellenlängenbereichs abstrahlt, das in der Lage ist, den in dem erfindungsgemäßen Mittel enthaltenen optischen Photosensibilisator zu aktivieren, so angeordnet ist, daß eine gleichmäßige Bestrahlung der Haarsträhnen gewährleistet ist. Die herausgezogenen Strähnen werden sodann mit einer für das Bleichen des Haares ausreichenden Lichtintensität bestrahlt.

Anschließend werden die die Lichtquelle enthaltende Haube und die für UV-Licht undurchlässige Strähnen-Folienhaube entfernt, das Haar mit einem zum Lösen des optischen Photosensibilisators geeigneten Lösungsmittel gespült und sodann getrocknet.

In einer besonderen Ausführungsform des erfindungsgemäßen Verfahrens wird das, gegebenenfalls mit Wasser angefeuchtete Haar zunächst mit 10 bis 120 g des erfindungsgemäßen Mittels durchtränkt, sodann das Haar mit einer für UV-Licht undurchlässigen, beispielsweise verspiegelten, Strähnen-Folienhaube bedeckt, die Haube mit einer, vorzugsweise dünnen, Häkelnadel mehrmals durchstochen, die jeweils darunterliegenden Haarsträhnen erfaßt und durch die Folie herausgezogen. Über den Kopf wird sodann eine für UV-Licht undurchlässige Haube gestülpt, in der eine Lichtquelle, welche Licht einer Wellenlänge oder eines Wellenlängenbereichs abstrahlt, das in der Lage ist, den in dem erfindungsgemäßen Mittel enthaltenen optischen Photosensibilisators zu aktivieren, so angeordnet ist, daß eine gleichmäßige Bestrahlung der Haarsträhnen gewährleistet ist. Das Haar wird sodann mit einer für das Bleichen des Haares ausreichenden Lichtintensität bestrahlt. Anschließend werden die die Lichtquelle enthaltende Haube und die für UV-Licht undurchlässige Strähnen-Folienhaube entfernt, das Haar mit einem zum Lösen des optischen Photosensibilisators geeigneten Lösungsmittel gespült und sodann getrocknet.

Gegebenenfalls kann das Haar bei dem erfindungsgemäßen Verfahren vor dem Trocknen gewaschen werden. Es ist ferner möglich, sofern nur die herausgezogenen Strähnen mit dem optischen Photosensibilisator durchtränkt sind, vor der Entfernung der Folien-Strähnenhaube, nur die Haarsträhnen mit dem Lösungsmittel zu spülen.

Das Bleichen des Haares kann auch mit einem Lichtkamm erfolgen. In dieser Ausführungsform des erfindungsgemäßen Verfahrens wird in der Verfahrensstufe (b) als Bestrahlungsquelle ein, nur an den Innenseiten der Kammzinken Licht abstrahlenden Lichtkamm verwendet, in den das abzugebende Licht, zum Beispiel durch Lichtleitfasern, von einer geeigneten Lichtquelle eingespeist wird.

Gegenstand der vorliegenden Erfindung ist zudem ein Mittel zum Bleichen der Haare unter Lichteinwirkung, welches als Lichtkamm vorgesehen ist, dessen Licht zwischen die Kammzinken, parallel zum Kammrücken beziehungsweise auf den Kammrücken zu, abgegeben wird.

Durch die Abgabe des Lichts zwischen den Kammzinken und parallel zum Kammrücken oder auf den Kammrücken zu wird selektiv das sich zwischen den Zähnen des Kammes befindende, zu bleichende Haar und nicht die Kopfhaut bestrahlt.

Die Ausstrahlrichtungen des Lichts sollen bei dem erfindungsgemäßen Lichtkamm bevorzugt in der Ebene liegen, die von den Kammzinken und dem Kammrücken aufgespannt wird. Der erfindungsgemäße Lichtkamm kann mehrere Lichtauslasse aufweisen, die zwischen benachbarten Kammzinken vorgesehen sind.

An einem Ausführungsbeispiel des erfindungsgemäßen Lichtkammes sind flexible Lichtleiter vorgesehen, die Lichtleitfasern sein können. Am Lichtkamm ist eine flexible Hülse zur Aufnahme eines Lichtleiterfaserkabels vorgesehen sein. Diese Hülse schützt zum einen die in ihr gelagerten Lichtleitfasern und vereinfacht zum anderen die Handhabung des Lichtkamms. Der flexible Lichtleiter kann aber auch als flexibler Schlauch ausgebildet sein, der mit einer für die Lichtleitung geeigneten Flüssigkeit gefüllt ist.

Die Verteilung des in den erfindungsgemäßen Lichtkamm eingetretenen Lichtes auf die einzelnen Kammzinken kann so erfolgen, daß die in jedem Zwischenraum zwischen den Kammzinken vorhandene Lichtintensität gleich groß ist. Dies kann durch eine gleichmäßige Verteilung des Lichtes auf die einzelnen Zinken mittels Lichtleitfasern erfolgen. In vorteilhafter Weise kann die gleichmäßige Lichtverteilung aber auch mittels teilweise durchlässiger Spiegel erfolgen. Entsprechende beispielhafte Ausführungsformen der Lichtverteilung innerhalb des Kammes werden nachfolgend im Beispiel B aufgeführt.

**Beispiele**

**Beispiel A: Verfahren zum Bleichen der Haare**

Eine mittelblonde menschliche Haarsträhne wird senkrecht in ein Quarzgefäß, das soweit mit der nachfolgenden Lösung gefüllt ist, daß die Haarspitzen in die Lösung eintauchen, gehängt:

```
    3 g Benzophenon
   97 g Ethanol (99,7 %ig)
  100 g
```

Das Licht einer 150 W Xenon-Niederdruck-Lichtbogenlampe (Modell C 9515 von Cathodean, Cambridge) wird auf die Haarsträhne fokussiert. Die Haarsträhne wird 1 h lang mit einer Lichtintensität von 4 W cm$^{-2}$ und einem Wellenlängenbereich von 300 bis 850 nm bestrahlt. Anschliessend wird die Haarsträhne aus dem Tank genommen, mit Ethanol ausgespült und getrocknet. Die ursprünglich mittelblonde Haarsträhne ist hellblond gebleicht.

**Beispiel B: Lichtkamm**

Im folgenden wird der Lichtkamm anhand von ein Ausführungsbeispiel darstellenden Figuren näher beschrieben. Es zeigt

Figur 1 in einer Seitenansicht mit teilweisem Vertikalschnitt einen Lichtkamm, der mittels eines Lichtleitfaserkabels mit Licht versorgt wird,

Figur 2 in einer perspektivischen Darstellung mit teilweisem Vertikalschnitt einen Teil des Lichtkamms nach Figur 1 mit geradlinig an den Kammzinken angeordneten Lichtauslassen, in die einzelne Lichtleitfasern münden, sowie

Figur 3 in einer Seitenansicht mit teilweisem Vertikalschnitt ein weiteres Ausführungsbeispiel eines Lichtkammes, in dessen hohlen Kammrücken teilweise durchlässige Spiegel hintereinander angeordnet sind, um jeweils Licht über Reflektoren in einzelne Lichtaustrittspalte zu lenken.

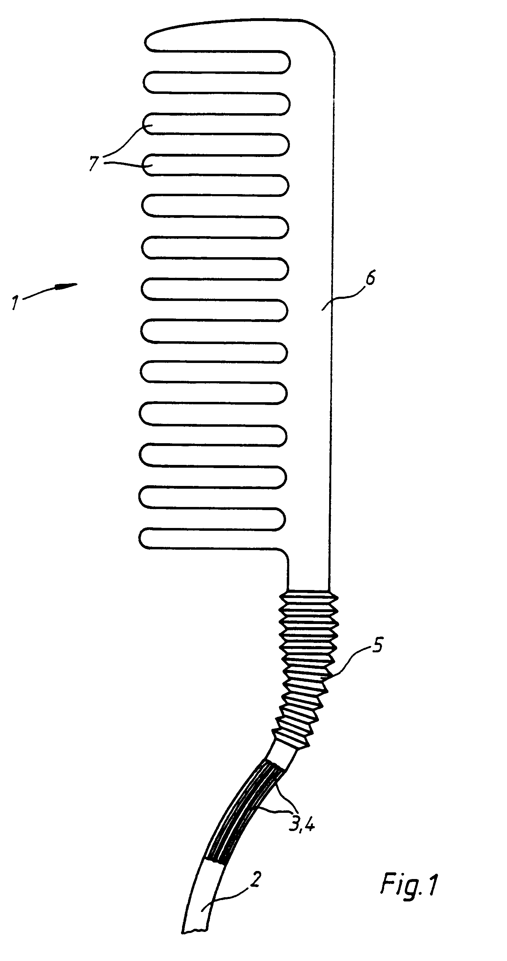

Der Lichtkamm 1 hat im wesentlichen die Form eines Frisierkammes (Figur 1). Er wird über ein Lichtleitfaserkabel 2 mit dem Licht einer nicht dargestellten Lichtquelle versorgt. Das Lichtleitfaserkabel 2 enthält als Lichtleiter 3 einzelne flexible Lichtleitfasern 4, die durch eine flexible Hülse 5 in den hohlen Kammrücken 6 geführt werden und sich dann auf die hohlen Kammzinken 7 verteilen.

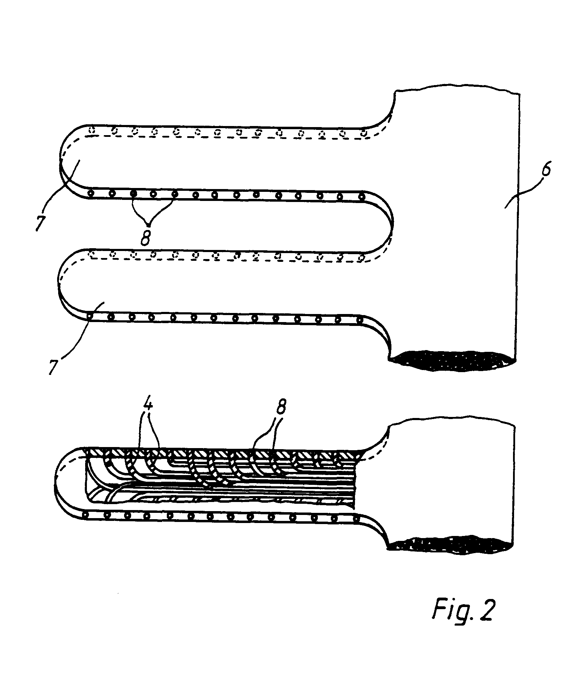

Die Lichtleitfasern 4 münden in Lichtauslasse 8, die in geradlinigen Reihen an den Kammzinken 7 derart angeordnet sind (Figur 2), daß das Licht nur parallel zum Kammrücken 6 in die vom Lichtkamm 1 aufgespannte Ebene abgestrahlt wird, um bei einer Haarbehandlung möglichst nur das Haar selbst zu bestrahlen.

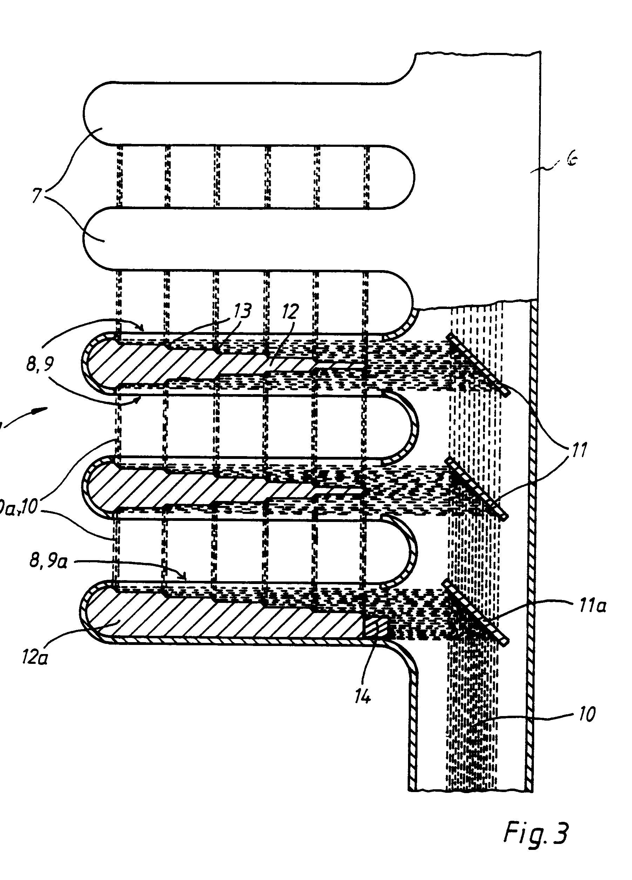

In Figur 3 ist ein Lichtkamm 1 dargestellt, bei dem als Lichtauslasse 8 Lichtaustrittsspalte 9, 9a vorgesehen sind. Das Licht 10 aus dem flexiblen Lichtleiter 3 wird mittels teilweise durchlässiger Spiegel 11, 11a und Reflektoren 12, 12a in die Lichtaustrittsspalte 9, 9a gelenkt. Um die gesamte Lichtleistung anteilsgleich auf die einzelnen Lichtaustrittsspalte 9, 9a aufzuteilen, ist die jeweilige Lichtdurchlässigkeit eines Spiegels 11, 11a derart gewählt, daß dieser bei insgesamt n Spiegeln den n-ten Teil der eingestrahlten Lichtleistung auf den ihm benachbarten Lichtaustrittsspalt 9a bzw. die ihm benachbarten zwei Lichtspalte 9, lenkt. Dies ist der Fall, wenn der n-te Spiegel 11, 11a vom flexiblen Lichtleiter 3 aus gezählt, bei insgesamt x Spiegeln 11, 11a eine Lichtdurchlässigkeit von $\dfrac{x-n}{x-(n-1)}$ aufweist.

Reflektoren 12, 12a mit stufenartigen Oberflächen lenken das Licht um einen Winkel von 90 ° um, so daß lediglich parallel zum Kammrücken 6 Licht zwischen die Kammzinken abgestrahlt wird.

Durch die Verkleinerung der am Reflektor 12, 12a tatsächlich reflektierenden, um 45 °C zum auftretenden Lichtstrahl angeordneten Flächen 13 kann deren Anzahl auf einem Reflektor 12, 12a erhöht werden. Dadurch wird die Anzahl der Strahlenbündel 10, 10a ebenfalls erhöht und man erreicht eine nahezu gleichmäßige Lichtabstrahlung zwischen den Kammzinken 7.

Das vom ersten Spiegel 11a ausgeblendete Licht wird zur Hälfte auf einen Lichtabsorber 14 gestrahlt und dort in Wärme umgewandelt. Diese Wärmemenge ist relativ klein.

Anstatt der beschriebenen Reflektoren 12, 12a können auch sonstige Reflektoren, zum Beispiel Hohlspiegel, in die Kammzinken eingesetzt werden. Ein in die Spitze eines Kammzinkens 7 eingesetzter Hohlspiegel kann den Zwischenraum zwischen zwei Kammzinken 7 besonders gleichmäßig bestrahlen. Der ausgestrahlte Winkelbereich erstreckt sich dann von einer durch die Spitzen der Kammzinken 7 verlaufenden Gerade bis zu den Kantenlinien der Spalte 9, 9a.

## Patentansprüche

1. Verfahren zum Bleichen der Haare unter Lichteinwirkung, bei dem man

   a) zunächst 10 bis 120 g eines in Form einer Lösung, einer Emulsion oder eines Gels vorliegenden, mindestens einen optischen Photosensibilisator und eine Verbindung, die in der Lage ist, ein Wasserstoff-Radikal zur Verfügung zu stellen, enthaltenden Mittels auf die Haare aufträgt und bis zu 60 Minuten lang einwirken läßt,

   b) sodann das Haar mit sichtbarem Licht und/oder UV-Licht einer Wellenlänge oder eines Wellenlängenbereichs, das in der Lage ist, den enthaltenen optischen Photosensibilisator zu aktivieren, mit einer für das Bleichen des Haares ausreichenden Intensität bestrahlt,

   c) anschließend das Haar gründlich spült und sodann trocknet.

2. Verfahren nach Anspruch 1, dadurch gekennzeichnet, daß das Haar vor dem Auftragen des den optischen Photosensibilisator enthaltenden Mittels mit Wasser angefeuchtet wird.

3. Verfahren nach Anspruch 1 oder 2, dadurch gekennzeichnet, daß das Haar mit Licht einer Lichtquelle, die ausgewählt ist aus Lichtbogenlampen, einer Deuterium-Lampe, einer Quecksilberdampf-Lampe, einer Xenon-Lampe, einem Halbleiterlaser, einem Stickstoff-Laser oder einem Helium-Cadmium-Laser, bestrahlt wird.

4. Verfahren nach einem der Ansprüche 1 bis 3, dadurch gekennzeichnet, daß die verwendete Lichtquelle im kontinuierlichen Modus betrieben wird.

**5.** Verfahren nach einem der Ansprüche 1 bis 4, dadurch gekennzeichnet, daß es sich um ein Verfahren zur Erzeugung heller Strähnen im Haar handelt.

**6.** Verfahren nach Anspruch 5, dadurch gekennzeichnet, daß man

a) das Haar, mit einer an sich bekannten Strähnen-Folienhaube bedeckt, anschließend die Haube mit einer Häkelnadel mehrmals durchsticht, die jeweils darunterliegenden Haarsträhnen erfaßt und durch die Folie herauszieht, auf diese Haarsträhnen 50 bis 100 g des Mittels nach einem der Patentansprüche 11 bis 15 aufträgt und dort bis zu 60 Minuten lang einwirken läßt,

b) sodann die herausgezogenen Strähnen mit Licht einer Wellenlänge oder eines Wellenlängenbereichs, das in der Lage ist, den in dem erfindungsgemäßen Mittel enthaltenen optischen Photosensibilisator zu aktivieren, mit einer für das Bleichen des Haares ausreichenden Intensität bestrahlt,

c) anschließend die Strähnen-Folienhaube entfernt, das Haar mit einem zum Lösen des optischen Photosensibilisators geeigneten Lösungsmittels spült und sodann trocknet.

**7.** Verfahren nach Anspruch 5, dadurch gekennzeichnet, daß man

a) das Haar mit 10 bis 120 g des Mittels nach einem der Patentansprüche 11 bis 15 durchtränkt, sodann mit einer für UV-Licht undurchlässigen Strähnen-Folienhaube bedeckt, die Haube mit einer Häkelnadel mehrmals durchsticht, die jeweils darunterliegenden Haarsträhnen erfaßt und durch die Folie herauszieht,

b) sodann die herausgezogenen Strähnen mit Licht einer Wellenlänge oder eines Wellenlängenbereichs, das in der Lage ist, den in dem erfindungsgemäßen Mittel enthaltenen optischen Photosensibilisator zu aktivieren, mit einer für das Bleichen des Haares ausreichenden Intensität bestrahlt,

c) anschließend die Strähnen-Folienhaube entfernt, das Haar gründlich mit einem zum Lösen des optischen Photosensibilisators geeigneten Lösungsmittel spült und sodann trocknet.

**8.** Verfahren nach Anspruch 5, dadurch gekennzeichnet, daß man

a) das Haar, mit einer für UV-Licht undurchlässigen Strähnen-Folienhaube bedeckt, anschließend die Haube mit einer Häkelnadel mehrmals durchsticht, die jeweils darunterliegenden Haarsträhnen erfaßt und durch die Folie herauszieht, auf diese Haarsträhnen 50 bis 100 g des Mittels nach einem der Patentansprüche 11 bis 15 aufträgt und dort bis zu 60 Minuten lang einwirken läßt,

b) sodann eine für UV-Licht undurchlässige Haube über den Kopf stülpt, in der eine Lichtquelle, welche Licht einer Wellenlänge oder eines Wellenlängenbereichs abstrahlt, das in der Lage ist, den in dem erfindungsgemäßen Mittel enthaltenen optischen Photosensibilisator zu aktivieren, so angeordnet ist, daß sie eine gleichmäßige Bestrahlung der Haarsträhnen gewährleistet, die herausgezogenen Strähnen mit einer für das Bleichen des Haares ausreichenden Lichtintensität bestrahlt,

c) anschließend die die Lichtquelle enthaltende Haube und die für UV-Licht undurchlässige Strähnen-Folienhaube entfernt, das Haar mit einem zum Lösen des optischen Photosensibilisators geeigneten Lösungsmittel spült und sodann trocknet.

**9.** Verfahren nach Anspruch 5, dadurch gekennzeichnet, daß man

a) das Haar mit 10 bis 120 g des Mittels nach einem der Patentansprüche 11 bis 15 durchtränkt, sodann das Haar mit einer für UV-Licht undurchlässigen Strähnen-Folienhaube bedeckt, die Haube mit einer Häkelnadel mehrmals durchsticht, die jeweils darunterliegenden Haarsträhnen erfaßt und durch die Folie herauszieht,

b) sodann eine für UV-Licht undurchlässige Haube über den Kopf stülpt, in der eine Lichtquelle, welche Licht einer Wellenlänge oder eines Wellenlängenbereichs abstrahlt, das in der Lage ist, den in dem erfindungsgemäßen Mittel enthaltenen optischen Photosensibilisator zu aktivieren, so angeordnet ist, daß eine gleichmäßige Bestrahlung der Haarsträhnen gewährleistet ist, die herausgezogenen Strähnen mit einer für das Bleichen des Haares ausreichenden Lichtintensität bestrahlt,

c) anschließend die die Lichtquelle enthaltende Haube und die für UV-Licht undurchlässige Strähnen-Folienhaube entfernt, das Haar mit einem zum Lösen des optischen Photosensibilisators geeigneten Lösungsmittel spült und sodann trocknet.

**10.** Verfahren nach einem der Ansprüche 1 bis 5, dadurch gekennzeichnet, daß die in der Verfahrensstufe (b) verwendete Bestrahlungsquelle ein nur an den Innenseite der Kammzinken Licht abstrahlender Lichtkamm ist.

**11.** Mittel zum Bleichen der Haare unter Lichteinwirkung zur Verwendung nach dem vorstehend beschriebenen Verfahren in Form einer Lösung, einer Emulsion oder eines Gels mit einem Gehalt an mindestens einer Verbindung, die in der Lage ist, ein Wasserstoff-Radikal zur Verfügung zu stellen, dadurch gekennzeichnet, daß es frei von Peroxiden ist und mindestens einen optischen Photosensibilisator enthält, der ausgewählt ist aus Benzophenon, 3-Brom-benzophenon, 4-Brombenzophenon, 2,5-Dihydroxyacetophenon, Benzil, p-Benzochinon, 2,3-Butadion, Michlers Keton, Acetophenon, 4-Benzylbenzoesäure, Methoxyacetophenon und 9-Fluorenon.

12. Mittel nach Anspruch 11, dadurch gekennzeichnet, daß es 0,1 bis 10 Gewichtsprozent mindestens eines optischen Photosensibilisators enthält.

13. Mittel nach einem der Ansprüche 11 oder 12, dadurch gekennzeichnet, daß der enthaltene optische Photosensibilisator durch sichtbares Licht und/oder UV-Licht aktiviert werden kann.

14. Mittel nach einem der Ansprüche 11 bis 13, dadurch gekennzeichnet, daß die enthaltene Verbindung, die in der Lage ist, ein Wasserstoff-Radikal zur Verfügung zu stellen, ein Alkohol ist.

15. Mittel nach Anspruch 14, dadurch gekennzeichnet, daß der Alkohol ausgewählt ist aus Ethanol, Methanol, Isopropanol, Cyclopentanol, Benzylalkohol, Cyclohexanol und Glykol.

16. Mittel zur Lichtbleichung von Haaren, dadurch gekennzeichnet, daß als Mittel ein Lichtkamm (1) vorgesehen ist, dessen Licht zwischen die Kammzinken (7) und parallel zum Kammrücken (6) oder auf den Kammrücken (6) zu abgestrahlt wird.

17. Mittel nach Anspruch 16, dadurch gekennzeichnet, daß die Ausstrahlrichtungen des Lichts in der Ebene liegen, die von den Kammzinken (7) und dem Kammrücken (6) aufgespannt wird.

18. Mittel nach Anspruch 17, dadurch gekennzeichnet, daß mehrere Lichtauslässe (8) im wesentlichen entlang einer geraden Linie am Kammzinken (7) angeordnet sind.

19. Mittel nach Anspruch 16, dadurch gekennzeichnet, daß am Lichtkamm (1) flexible Lichtleiter (3) vorgesehen sind.

20. Mittel nach Anspruch 19, dadurch gekennzeichnet, daß die Lichtleiter (3) Lichtleitfasern (4) sind.

21. Mittel nach den Ansprüchen 18 und 20, dadurch gekennzeichnet, daß die einzelnen Lichtleitfasern (4) in die einzelnen Lichtauslasse (8) münden.

22. Mittel nach Anspruch 20, dadurch gekennzeichnet, daß zwischen benachbarten Kammzinken (7) ein Lichtaustrittsspalt (9, 9a) als Lichtauslaß (8) vorgesehen ist.

23. Mittel nach Anspruch 22, dadurch gekennzeichnet, daß das aus dem flexiblen Lichtleiter (3) ausgetretene Licht (10, 10a) mittels eines teilweise durchlässigen Spiegels (11, 11a) über einen Reflektor (12, 12a) in einen Lichtaustrittsspalt (9, 9a), gelenkt wird.

24. Mittel nach Anspruch 23, dadurch gekennzeichnet, daß der n-te Spiegel (11, 11a) vom flexiblen Lichtleiter (3) aus gezählt, bei insgesamt x Spiegeln (11, 11a) im Lichtkamm (1) eine Lichtdurchlässigkeit von

$$\frac{x-n}{x-(n-1)}$$ aufweist.

25. Mittel nach Anspruch 23, dadurch gekennzeichnet, daß ein überschüssiger Lichtanteil auf einen Lichtabsorber (14) gestrahlt wird.

26. Mittel nach Anspruch 20, dadurch gekennzeichnet, daß am Lichtkamm (l) eine flexible Hülse (5) zur Aufnahme eines Lichtleitfaserkabels (2) vorgesehen ist.


**Claims**

1. Method of bleaching hair under the influence of light, wherein
   a) firstly 10 to 120 g of an agent which is either a solution, an emulsion or a gel and contains at least one optical photosensitizer and a compound which is capable of providing a hydrogen radical, is applied onto the hair and is left to act for up to 60 minutes,
b) subsequently the hair is irradiated with visible light and/or UV light of a wavelength or wavelength range which is capable of activating the contained photosensitizer, and at an intensity sufficient to bleach the hair.
c) subsequently the hair is thoroughly rinsed and then dried.

2. Method according to claim 1, characterised in that the hair is moistened with water before the agent containing the optical photosensitizer is applied.

3. Method according to claims 1 or 2, characterised in that the hair is irradiated with light from a light source which is selected from arc lamps, a deuterium lamp, a mercury vapour lamp, a xenon lamp, a semiconducter laser, a nitrogen laser or a helium-cadmium laser.

4. Method according to any one of claims 1 to 3, characterised in that the light source used is operated in a continuous mode.

5. Method according to any one of claims 1 to 4 characterised in that it is a method of generating light strands in the hair.

6. Method according to claim 5, characterised in that
a) the hair is covered with a strand foil cap know per se, subsequently the cap is pierced several times with a crochet hook, the hair strands underneath in each case are pulled out through the foil, 50 to 100 g of the agent according to any one of claims 11 to 15 are applied to these hair strands and are left thereon to act for up to 60 minutes,
b) subsequently the pulled out strands are irradiated with light of a wavelength or wavelength range which is capable of activating the optical photosensitizer which is present in the agent according to the invention, its intensity being sufficient to bleach the hair,
c) subsequently the strand-foil cap is removed, the hair is rinsed with a solvent which is suitable for dissolving the optical photosensitizer, and then dried.

7. Method according to claim 5, characterised in that
a) the hair is wetted with 10 to 120 g of the agent according to any one of claims 11 to 15, subsequently it is covered with a strand foil cap which is impermeable to UV light, the cap is pierced several times with a crochet hook, the hair strands underneath in eache case are caught and pulled out through the foil,
b) subsequently the pulled out strands are irraddiated with light of a wavelength or wavelength range which is capable of activating the optical photosensitizer which is present in the agent according to the invention, its intensity being sufficient to bleach the hair.
c) subsequently the strand foil cap is removed, the hair is thoroughly rinsed with a solvent which is suitable for dissolving the optical photosensitizer, and then dried.

8. Method according to claim 5, characterised in that
a) the hair is covered with a strand foil cap which is impermeable to UV light, subsequently the cap is pierced several times with a crochet hook, the hair strands underneath in each case are caught and pulled out trough the foil, 50 to 100 g of the agent according to any one of claims 11 to 15 are applied onto these strands and are left to act for up to 60 minutes;
b) subsequently a cap is pulled over the head which cap is impermeable to UV light and in which a light source, which emits light of a wavelength or wavelength range which is capable of activating the optical photosensitizer which is present in the agent according to the invention, is disposed in such a manner that a uniform irradiation of the hair strands is ensured, and irradiates the pulled out strands at a light intensity which is sufficient to bleach the hair,
c) subsequently the cap which contains the light source and the strand foil cap which is impermeable to UV light are removed, the hair is rinsed with a solvent which is suitable for dissolving the optical photosensitizer and then dried.

9. Method according to claim 5, characterised in that
a) the hair is wetted with 10 to 120 g of the agent according to any one of claims 11 to 15, subsequently the hair is covered with a strand foil cap which is impermeable to UV light, the cap is pierced several times by means of a crochet hook, the hair strands underneath in each case are caught and pulled out through the foil,

b) subsequently a cap is pulled over the head which cap is impermeable to UV light and in which a light source which emits light of a wavelength or wavelength range which is capable of activating the optical photosensitizer which is present in the agent according to the invention, is disposed in such a manner that uniform irradiation of the hair strands is ensured, the pulled out strands are irradiated at a light intensity which is sufficient to bleach the hair,

c) subsequently the cap containing the light source and the strand foil cap which is impermeable to UV light are removed, the hair is rinsed with a solvent which is suitable for dissolving the optical photosensitizer, and then dried.

10. Method according to any one of claims 1 to 5, characterised in that the radiation source used in method stage (b) is a light comb which emits light only on the inner side of the comb teeth.

11. Agent for bleaching hair unter the influence of light to be used according to the above-described method in the form of a solution, an emulsion or gel containing a least one compound which is capable of providing a hydrogen radical, characterised in that it is free of peroxides and contains at least one optical photosensitizer selected from benzophenone, 3-bromobenzophenone, 4-bromobenzophenone, 2,5-dihydroxyacethophenone, benzil, p-benzoquinone, 2,3-butanedione, Michler's ketone, acetophenone, 4-benzylbenzoic acid, methoxyacetophenone and 9-fluorenone.

12. Agent according to claim 11, characterised in that it contains 0,1 to 10 percent by weight of the optical photosensitizer.

13. Agent according to any ohne of claims 11 or 12, characterised in that the optical photosensitizer may be activated by visible light and/or UV light.

14. Agent according to any one of claims 11 to 13, characterised in that the compound which is capable of providing a hydrogen radical is an alcohol.

15. Agent according to claim 14, characterised in that the alcohol is selected from ethanol, methanol, isopropanol, cyclopentanol, benzyl alcohol, cyclohexanol and glycol.

16. Agent for light-bleaching hair, characterised in that as agent a light comb (1) is provided, the light from which is emitted between the comp teeth (7) and parallel to the comb back (6) or onto the comb back (6).

17. Agent according to claim 16, characterised in that the directions of light emission lie in the plane which is spannend by the comb teeth (7) and the comb back (6).

18. Agent according to claim 17, characterised in that several light openings (8) are disposed essentially along a straight line at the comb teeth (7).

19. Agent according to claim 16, characterised in that flexible light conductors (3) are provided at the light comb (1).

20. Agent according to claim 19, characterised in that the light conductors (3) are light conducting fibres (4).

21. Agent according to claims 18 and 20, characterised in that the individual light conducting fibres (4) open into the individual light openings (8).

22. Agend according to claim 20, characterised in that between the adjacent comb teeth (7) a light emission gap (9, 9a) is provided as light outlet (8).

23. Agent according to claim 22, characterised in that the light (10, 10a) emerging from the flexible light conductor (3) is guided into a light emission gap (9, 9a) via a reflector (12, 12a) by means of a mirror (11, 11a) which is partially light-permeable.

24. Agent according to claim 23, characterised in that the nth mirror (11, 11a) counted from the flexible light conductor (3) shows a light permeability of

$$\frac{x - n}{x - (n - 1)}$$

in the light comb (1) for a total of x mirrors (11, 11a).

**25.** Agent according to claim 23, characterised in that excess light is emitted to a light absorber (14).

**26.** Agent according to claim 23, characterised in that at the light comb (1) a flexible sleeve (5) is provided for receiving a light conducting fibre cable (2).


**Revendications**

**1.** Méthode de décoloration des cheveux sous l'effet de la lumière, dans lequel:
a) on applique d'abord sur les cheveux 10 à 120 g d'un produit sous la forme d'une solution, d'une émulsion ou d'un gel avec un teneur en au moins un photo-sensibilisateur optique appliqué et un un composé, qui est en mesure de fournir un radical hydrogène, et l'on laisse agir jusqu'à 60 minutes,
b) on irradie ensuite les cheveux, avec une intensité suffisante pour obtenir leur décoloration, à la lumière visible et/ou à la lumière UV, d'une longueur d'onde ou d'une plage de longueur d'onde qui est en mesure d'activer le photo-sensibilisateur optique,
c) immédiatement après, on rince à fond les cheveux et on les sèche.

**2.** Méthode selon la revendication 1, caractérisée en ce que les cheveux sont humidifiés à l'eau, avant application du produit contenant le photo-sensibilisateur optique.

**3.** Méthode selon la revendication 1 ou 2, caractérisée en ce que l'on irradie les cheveux avec une lumière provenant d'une source lumineuse, sélectionnée parmi les lampes à arc, au deutérium, à vapeur de mercure, au xénon, un laser à semiconducteur, un laser à l'azote ou un laser hélium-cadmium.

**4.** Méthode selon l'une des revendications 1 à 3, caractérisée en ce que la source lumineuse utilisée est exploitée en mode continu.

**5.** Méthode selon l'une des revendications 1 à 4, caractérisée en ce qu'il s'agit d'une méthode pour produire des mèches claires dans les cheveux.

**6.** Méthode selon la revendication 5, caractérisée en ce que
a) l'on recouvre les cheveux avec un capuchon en feuille de méchage connu en soi, immédiatement après, l'on perce en plusieurs endroits le capuchon à l'aide d'un crochet à dentelle, on saisit les mèches de cheveux se trouvant au-dessous de chaque percement et on les sort au travers de la feuille, on applique sur ces mèches de cheveux de 50 à 100 g de produit selon les revendications 11 à 15, et l'on laisse agir ainsi pendant une durée allant jusqu'à 60 minutées,
b) ensuite, on irradie, avec une intensité suffisante pour obtenir la décoloration des cheveux, les mèches sorties à l'aide de lumière d'une longueur d'onde ou d'une plage de longueur d'onde qui est en mesure d'activer le photo-sensibilisateur optique contenu dans le produit selon l'invention,
c) immédiatement après, on enlève le capuchon en feuille de mèchage, on rince les cheveux à l'aide d'un solvant convenant pour dissoudre le photo-sensibilisateur optique, et ensuite l'on sèche.

**7.** Méthode selon la revendication 5, caractérisée en ce que
a) on imprègne les cheveux à l'aide de 10 à 120 g du produit selon l'une des revendications 11 à 15, ensuite l'on recouvre d'un capuchon à feuille de méchage imperméable à la lumière UV, on perce en plusieurs endroits le capuchon à l'aide d'un crochet à dentelle, on saisit les mèches qui se trouvent chaque fois sous le percement et on les sort par la feuille,
c) immédiatement après, on enlève le capuchon en feuille de méchage, on rince à fond les cheveux à l'aide d'un solvant convenant pour dissoudre le photo-sensibilisateur optique, et ensuite l'on séche.

**8.** Méthode selon la revendication 5, caractériée en ce que
a) on recouvre les cheveux à l'aide d'un capuchon à feuille de méchage imperméable à la lumière UV, on perce ensuite en plusieurs endroits le capuchon à l'aide d'un crochet à dentelle, on saisit les méches qui se trouvent chaque fois sous le percement et on les sort par la feuille, on applique sur ces mèches de cheveux de 50 à 100 g du produit selon l'une des revendications 11 à 15, et l'on laisse agir pendant une durée allant jusqu'à 60 minutes.
b) ensuite, on passe sur la téte un capuchon imperméable à la lumière UV, dans lequel une source lumineuse, qui irradie une lumière d'une longueur d'onde ou d'une plage de longueur d'onde qui est en mesure d'activer le photo-sensibilisateur optique contenu dans le produit selon l'invention, est disposée de telle facon qu'elle assure une irradiation régulière des mèches de cheveux, en irradiant les mèches sorties avec une intensité lumineuse suffisante pour obtenir la décoloration des cheveux,

c) immédiatement après, on enlève le capuchon contenant la source lumineuse et le capuchon en feuille de méchage imperméable à la lumière UV, on rince à fond les cheveux á l'aide d'un solvant convenant pour dissoudre le photo-sensibilisateur optique, et ensuite l'on sèche.

9. Méthode selon la revendication 5, caractérisée en ce que

a) on imprègne les cheveux avec 10 à 120 g du produit selon l'une des revendications 11 à 15, ensuite, on recouvre les cheveux à l'aide d'un capuchon à feuille de méchage imperméable à la lumière UV, on perce ensuite en plusieurs endroits le capuchon à l'aide d'un crochet à dentelle, on saisit les mèches qui se trouvent chaque fois sous le percement et on les sort par la feuille,

b) ensuite, on passe sur la téte un capuchon imperméable à la lumière UV, dans lequel une source lumineuse, qui irradie une lumière d'une longueur d'onde ou d'une plage de longueur d'onde qui est en mesure d'activer le photo-sensibilisateur optique contenu dans le produit selon l'invention, est disposée de telle facon qu'elle assure une irradiation régulière des mèches de cheveux, en irradiant les mèches sortie avec une intensité lumineuse suffisante pour obtenir la décoloration des cheveux,

c) immédiatement après, on enlève le capuchon contenant la source lumineuse et le capuchon en feuille de méchage impérméable à la lumière UV, on rince à fond les cheveux à l'aide d'un solvant convenant pour dissoudre le photo-sensibilisateur optique, et ensuite l'on sèche.

10. Méthode selon l'une des revendications 1 à 5, caractérisée en ce que la source lumineuse utilisée à l'étape (b) de la méthode est un peigne lumineux, irradiant de la lumière seulement sur les faces inférieures de ses dents.

11. Produit de décoloration des cheveux sous l'effet de la lumière, par utilisation de la méthode décrite ci-dessus, sous forme d'une solution, d'une émulsion ou d'un gel, avec une teneur en au moins un composé, qui est en mesure de fournir un radical hydrogène, caractérisé en ce qu'il ne contient pas des peroxydes et en ce qu'il contient au moins un photo-sensibilisateur optique sélectionné parmi la benzophénone, la 3-brome-benzophénone, la 4-brome-benzophénone, la 2,5-dihydroxyacétophénone, le benzyle, la p-benzochinone, la 2,3-butandione, le cétone de Michler, l'acétophénone, l'acide 4-benzyl-benzoique, la méthoxyacétophénone et la 9-fluorénone.

12. Produit selon la revendication 11, caractérisé en ce qu'il contient de 0,1 à 10 % en poids du photo-sensibilisateur optique.

13. Produit selon l'une des revendications 11 ou 12, caractérisé en ce que le photo-sensibilisateur optique contenu peut étre activé par de la lumière visible et/ou de la lumière UV.

14. Produit selon l'une des revendications 11 à 13, caractérisé en ce que le composé contenu, qui est en mesure de fournir un radical hydrogène, est un alcool.

15. Produit selon la revendication 14, caractérisé en ce que l'alcool est sélectionné parmi l'éthanol, le méthanol, l'isopropanol, le cyclopentanol, l'alcool benzylique, le cyclohexanol et le glycol.

16. Produit pour la décoloration des cheveux, caractérisé en ce que l'on utilise comme instrument un peigne lumineux (1), dont la lumière irradie entre les dents de peigne (7) et parallèlement aux dos de peigne (6) ou en direction de ce dernier.

17. Produit selon la revendication 16, caractérisé en ce que les directions d'irradiation de la lumière sont situées dans le plan passant par les dents de peigne (7) et le dos de peigne (6).

18. Produit selon la revendication 17, caractérisé en ce que plusieurs sorties de lumière (8) sont disposées sur les dents de peigne (7) le long d'une ligne droite.

19. Produit selon la revendication 16, caractérisé en ce que des guides de lumière (3) flexibles sont prévues sur le peigne lumineux (1).

20. Produit selon la revendication 19, caractérisé en ce que les guides de lumière (3) sont des fibres optiques (4).

21. Produit selon les revendications 18 et 20, caractérisé en ce que les diférentes fibrex optiques (4) débouchent dans les différentes sorties de lumière (8).

22. Produit selon la revendication 20, caractérisé en ce qu'un interstice de sortie de lumière (9, 9a), servant de sortie de lumière (8), est prévu entre des dents de peigne (7) voisines.

23. Produit selon la revendication 22, caractérisé en ce que la lumière (10, 10a) qui sort du guide de lumière (3) flexible est déviée, au moyen d'un miroir (11, 11a) partiellement transparent, par l'intermédiaire d'un réflecteur (12, 12a), pour aller dans un interstice de sortie de lumière (9, 9a).

24. Produit selon la revendication 23, caractérisé en ce qu'en comptant depuis le guide de lumière (3) flexible, le nième miroir (11, 11a) présente une transparence de

$$\frac{x - n}{x - (n - 1)},$$

pour un total de x miroirs (11, 11a) dans le peigne lumineux (1).

25. Produit selon la revendication 23, caractérisé en ce qu'une proportion en excès de lumière est irradiée sur un absorbeur de lumière (14).

26. Produit selon la revendication 23, caractérisé en ce qu'une gaine flexible (5) servant à recevoir un câble à fibre optique (2) est prévue sur le peigne lumineux (1).

14

*Fig.1*

15

EP 0 451 261 B1

Fig. 2

16

Fig. 3